# EUROPEAN PATENT APPLICATION

(11) **EP 2 952 221 A1**
(43) Date of publication of application: **09.12.2015**
(21) Application number: 14745868.1
(22) Date of filing: 29.01.2014
(51) Int. Cl.: A61M 25/00

(54) **CLOSED PERIPHERAL INTRAVENOUS CATHETER WITH SAFETY SYSTEM CPIVCSS**

(30) Priority: 30.01.2013 MX 2013001219
(71) Applicant: Equipos Médicos Vizcarra, S.A., Emiliano Zapata, Morelos 62765 (MX)
(72) Inventor: ARELLANO CABRERA, José Antonio, Morelos C.P. 62765 (MX); RODRIGUEZ LELIS, José María, Morelos C.P. 62765 (MX); LUCAS JIMENEZ, María Teresa, Morelos C.P. 62765 (MX); CHALITA VIZCARRA, Alfredo, Morelos C.P. 62765 (MX)
(74) Representative: Temino Ceniceros, Ignacio
(86) International application number: PCT/MX2014/000033
(87) International publication number: WO 2014/119988

(57) **Abstract**

The present invention refers to a system for placing an intravenous catheter, specifically a closed system for placing an intravenous catheter which prevents exposure by medical personnel to the patient's bodily fluids during the placement of the catheter; and with a retractable needle cannula in a manual billows mechanism for preventing its reuse, accidental puncture wounds and assuring safe disposal. This CPIVCSS has the advantages of (a) preventing medical personnel from being exposed to the contaminated bodily fluids or blood during the placing of the catheter in the patient; (b) reducing the possibility of multiple punctures and; (c) having an easily usable safety system. The Closed Peripheral Intravenous Catheter with Safety System includes two systems, one the piercing and safety system made up of: (2) piercing cannula, (10) mounting body, (11) affixing body, (9) flexible billows tube, (8) flashback chamber and filter and (13) filter paper; and the closed peripheral intravenous catheter made up of: (3) the catheter cube or hub, (1) the catheter tube, (12) the hub seal, (4) the affixing wings, (5) the flow tube, (6) flow safety valve and (7) the multi-piercing connector.

## Description

### FIELD OF THE INVENTION

The present invention is related to the industry which manufactures hospital instruments. More specifically it is related to the industry which manufactures instruments for venous p and catheterization. Even more specifically, it is related to a catheter and needle assembly, which includes a protection unit for the needle which safely protects the far sharp end of a puncturing needle, after said needle has been used.

### BACKGROUND OF THE INVENTION

As Rivera and collaborators [1] mention, IV intravenous therapy is in answer to the need to transfer blood from one human being to another and it dates back to approximately 1492. In medicine, the assembly of a catheter and puncturing needle are used to effectively place a catheter into the circulatory system of a patient. There are essentially two kinds of assemblies for placing venous catheters within the circulatory system of a patient. The first is a catheter placed within a puncturing needle which is used to puncture the skin and place a catheter into the circulatory system of the patient, the needle is removed, leaving the catheter in the circulatory system of the patient. The second kind of unit for placing a catheter consists of a disposable needle which passes through the catheter in order to puncture a vein and allow for placing the catheter. Once placing of the assembly in the vein is checked using retrospection of blood in the needle, the needle is removed, leaving the intravenous catheter in place for its use in connecting an intravenous bag or bottle or a stopper for later use.

Between the time when the needle is removed and connecting the intravenous catheter to the flow supply line, blood from the patient's vein may drip from the catheter. Also blood usually remains in the used needle and this may also overflow or drip on the clinic personnel who are carrying out the procedure. Another risk factor associated with the use of these kinds of devices is the accidental puncturing of clinic personnel with the used needle and contamination with patient's blood or bodily fluids.

Throughout the world there is epidemiological evidence that the biggest worry related to the use of sharp instruments and their treatment as infectious waste in hospitals is the transmission of the AIDS virus and more frequently, Hepatitis B and C viruses. This is due to wounds caused by needles contaminated with human blood. There is then a growing need for producers of catheters to prevent accidental wounds with needles to medical personnel and other employees who handle hospital waste, wash the clothing or other residues which contain used needles. As a result, new products have been designed to incorporate special covers for the needle or mechanisms for retracting the needle inside a protective chamber. Such devices are described, for example in U.S. patents No. 4.747.831, 4.828.548, 5.129.884, 5.501.675, 5.817.058, 5.989.220, 7.771.394 and 7.740.615. Many of the devices described in these patents have various parts which substantially raise manufacturing costs, besides interfering in the ability of the user to sense when the needle is correctly inserted in the patient's vein. Other devices require two-handed handling or, due to the design of the retraction of the needle by activating a safe spring system, are prone to premature retraction of the needle while being transported, stores and handled.

Still another problem associated with peripheral type intravenous catheters is the dripping caused by overflow of blood coming from the opening of the catheter where the IV line is to be attached, when it is not attached. This overflow of dripping blood may come into contact with the clinic personnel and especially with the technician who carries out the catheterization process, putting that person in risk of contamination and the transmission of diseases, health problems and increases in the costs of healthcare systems.

As may be inferred from the text, there are certain qualities which an intravenous catheterization device should have. Among these are: a) to prevent exposure of medical personnel to contaminated fluids of blood during the process of placing the catheter in a patient; and b) to have an easily manageable safety system.

In a catheterization device with a quick and painless puncturing system, a means for protecting the needle or cannula while at the same time protecting the user and avoiding accidental injury with the tip is presented. However, in a catheter without a quick and painless puncturing system, there has been no system for protecting the needle as indicated in the present application.

As an example of the kind of protection mentioned in the preceding paragraph, we have Mexican application MX/a/2011/013382 of the same applicant as the present application. However, this device does not have a mechanism for preventing that upon removing the cannula or connecting the IV line there is a probability of contact by the handler with fluids or blood.

### OBJECTIVE OF THE INVENTION

The main objective of the invention is to achieve a device for intravenous catheterization which prevents exposure to contaminated bodily fluids by medical personnel during the placing and withdrawal of the catheter in the patient.

Still another objective is having a device which integrates a safety system which encapsulates the cannula with the sharp point, which is compact and easy to use.

Other objectives and advantages may become apparent through reading this application and the drawings which are included for strictly illustrative, non-limiting, ends and which form an integral part of the present description.

### BRIEF DESCRIPTION OF THE INVENTION

The present invention refers to a system for placing an intravenous catheter, especially a closed intravenous catheter placement system, which prevents exposure by medical personnel to a patient's bodily fluids during the placing of the catheter; and with a cannula for a retractable needle in a manual bellows mechanism to ensure that the needle cannot be reused, cause accidental puncture wounds and to ensure its safe disposal.

The present invention refers to a double system which allows, in one of its components, a closed peripheral intravenous catheter system, to avoid contact with a patient's bodily fluid during the catheterization process, besides being a closed system which reduces the possibility of the growth of microorganisms in the puncture area since the connections are multi-piercing connections and rapid connections which keep the inside ducts of the device through which fluids and medicine pass into the circulatory system, isolated from the external environ. In a second component, a manual safety mechanism which allows for the retrieval of the contaminated cannula inside a protective chamber after the piercing process, in order to avoid later contact of the users or other persons, thus preventing puncturing accidents and the transmission of diseases.

Likewise, the characteristics of the retraction system assure that there is no contact with the cannula after the catheter has been placed in the patient, and which allows it to be placed at a safe distance within the protective chamber of the safety system.

The CPIVCSS assembly of the present invention consists of a hollow catheter hub which on one end has a soft flexible stopper which serves as a seal and only allows the passage and withdrawal of the introductory needle and which on the other coaxial end the catheter which during catheterization is mechanically anchored within the patient's vein is found.

The hollow catheter hub on one side has an opening from which extends from its periphery another hollow hub at an angle greater than zero degrees and on whose free end a translucent flow tube is connected, extending the length of at least 3 cm. On the free end of the tube a system of multi-piercing connectors and quick connectors is connected which avoid the outflow of fluids from inside the device and only allow the inflow of fluids upon connecting the IV lines or injecting medications.

The hollow catheter hub, on the part opposite the placement of the catheter, has an affixing body which has an opening for the passage of the introducing needle. This first affixing body, in the first stage of CPIVCSS operation is to be used by the technician responsible for placing the catheter in the patient to hold the device and carry out the catheterization process. During the second stage of the CPIVCSS operation it is used to block the pointed end of the introducing needle and prevent accidental wounds.

Attached to the other end of the affixing body is a flexible bellows tube in which the introducing needle is housed when the CPIVCSS safely system is activated. The billows is initially located in a state of compression, while the other end of the billows is attached to the body of the housing which acts as a base for mounting the introducing needle. With this configuration the introducing needle passes through the flexible billows tube, from the affixing body, goes through the flexible top of the catheter hub and is placed inside the catheter. All of the components in the aforementioned configuration make up the CPIVCSS in the piercing modality.

Besides that, on one of the ends of the body of the housing is a translucent hollow cylinder or filter and flashback chamber, which allows the technician responsible for the catheterization procedure to see the flashback of the blood when the far away tip of the needle has been placed in the patient's vein. Once catheterization has taken place and the introducing needle along with the catheter are in the patient's vein, the technician responsible for carrying out the procedure, without moving the affixing body, pulls the body of the housing away from the catheterization area. This process pulls the introducing needle from the patient's vein, leaving the catheter in place; it also extends the billows to cover the part of the cannula which was in contact with tissues of the body, including the blood and encapsulating the introducing needle so that possible accidental puncture wounds or spillage of contaminated fluids from the patient does not occur.

### BRIEF DESCRIPTION OF THE DRAWINGS

Figure 1 shows an exploded view of the closed peripheral intravenous catheter with safety system CPIVCSS, showing the parts which make it up.
Figure 2 is an isometric view of the closed peripheral intravenous catheter with safety system CPIVCSS, showing how the fitting together of the security system and the closed peripheral intravenous catheter takes place.
Figure 3 shows an isometric view of the closed peripheral intravenous catheter with safety system CPIVCSS.
Figure 4 shows a side view of the section of the closed peripheral intravenous catheter with safety system CPIVCSS in the piercing stage.
Figure 5 shows an isometric view of the closed peripheral intravenous catheter with safety system CPIVCSS in the safety stage to prevent accidental puncture wounds and contact with the patient's contaminated fluids.
Figure 6 shows a side view of a section of the closed peripheral intravenous catheter with safety system CPIVCSS in the safety stage and a detailed view of the configuration for blocking the sharp ends of the introducing needle.
Figure 7 shows an isometric view of the closed peripheral intravenous catheter with safety system CPIVCSS in the safety stage and the disconnecting of the closed peripheral intravenous catheter.
Figure 8 shows a cross section view of the closed peripheral intravenous catheter with safety system CPIVCSS in the safety stage and the disconnecting of the closed peripheral intravenous catheter.

### DETAILED DESCRIPTION OF THE INVENTION

We use the figures of the preferred modality of the present invention for the detailed description.

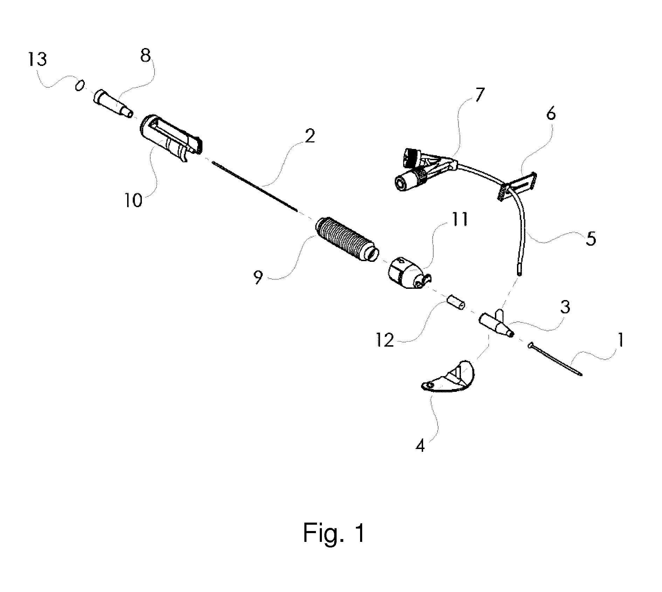

An exploded view of the thirteen elements which make up the closed peripheral intravenous catheter with safety system CPIVCSS is shown in Figure 1. As can be seen in this Figure 1, the parts which make up the CPIVCSS are: 1) the catheter tube, 2) the piercing cannula, 3) the catheter cube or hub, 4) the affixing wings, 5) the flow tube, 6) the valve to close the flow, 7) the multi-piercing connector, 8) flashback chamber and filter, 9) flexible billows tube, (10) mounting body, (11) affixing body, (12) hub seal and (13) filter paper.

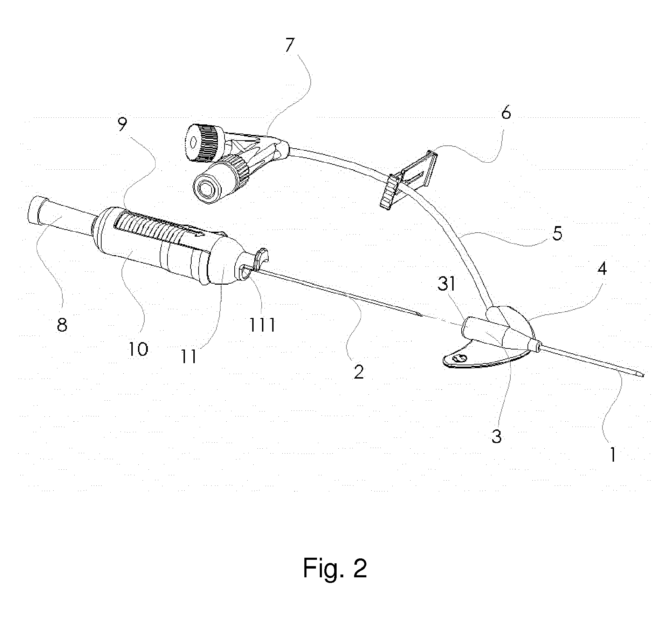

The isometric view of the closed peripheral intravenous catheter and the piercing and safety system in connecting mode is shown in Figure 2. The affixing body 11) joins the flexible billows tube 9) and this in turn connects to the mounting body (10) which supports the piercing cannula (2) which passes through the flexible billows tube (9) and the affixing body (11), while on the other end of the mounting body (10) the flashback chamber and filter (8) are attached. All of these elements make up the piercing and safety system. The piercing and safety system is attached to the closed peripheral intravenous catheter by way of a mounting opening (111) in the affixing body (1) and the sealed end (31) of the catheter hub (3).

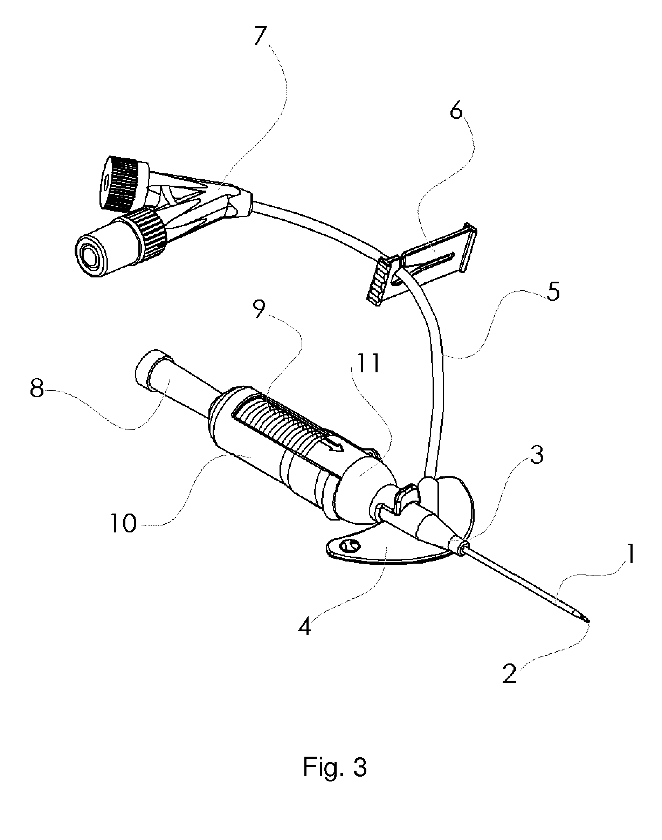

Once attached the safety and piercing system with the closed peripheral intravenous catheter forms the closed peripheral intravenous catheter with safety system CPIVCSS. An isometric view is shown in Figure 3. The CPIVCSS is made up of the mounting body (10) which adjoins the flexible billows tube (9) and the affixing body (11) to which is connected the catheter hub (3). The piercing cannula (2) is attached to the mounting body (10) and passes through the concentric center of the flexible billows tube (9) and the affixing body (11), penetrating the hub seal (12) in order to cross the catheter hub (3) and remains housed in the inside of the catheter tube (1) which is attached to the catheter hub (3) at one of its ends. On the side of the catheter hub (3) is connected the flow tube (5) which on its opposite end is connected a multi-piercing connector (7) and on top of the flow tube (5) is mounted the valve to close the flow.

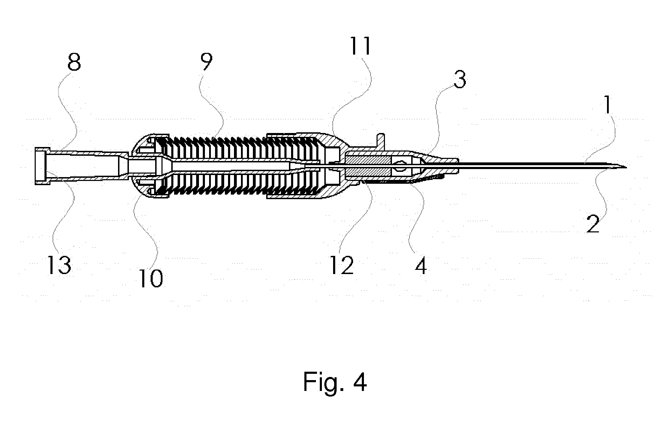

In the puncturing stage of the CPIVCSS as shown in the cross section in Figure 4, the piercing cannula (2) is connected to the affixing body (10) at one end while the other end is attached to the flashback chamber and filter (8), forming a canal which serves as passage of the blood signal when the introducing cannula penetrates the patient's vein and also a filter paper is placed (13) to avoid spillage of blood from the canal of the piercing system.

Attached to the affixing body (10) is the compressed flexible billows tube (9) which covers part of the piercing cannula (2), while on the other end of the flexible billows tube (9) the affixing body (11) is attached. The piercing cannula (2) passes through the affixing body (11) and penetrates the hub seal (12) and crosses the catheter hub (3) and remains housed in the inside of the catheter tube (1) which is attached to the catheter hub (3) at one of its ends. On the bottom of the catheter hub (3) are the affixing wings (4) which serve to affix the closed peripheral intravenous catheter to the piercing area on the patient's body.

During the catheterization process the medical personnel in charge of the procedure holds the CPIVCSS of the affixing body (11) and introduces the piercing cannula (2) along with the catheter tube (1) into the circulatory system of the patient. By way of blood retrospection in the flashback chamber and filter (8) the medical personnel assures the correct placement of the closed intravenous catheter into the circulatory system of the patient and proceeds to withdraw the piercing cannula (2), thus activating the CPIVCSS safety system.

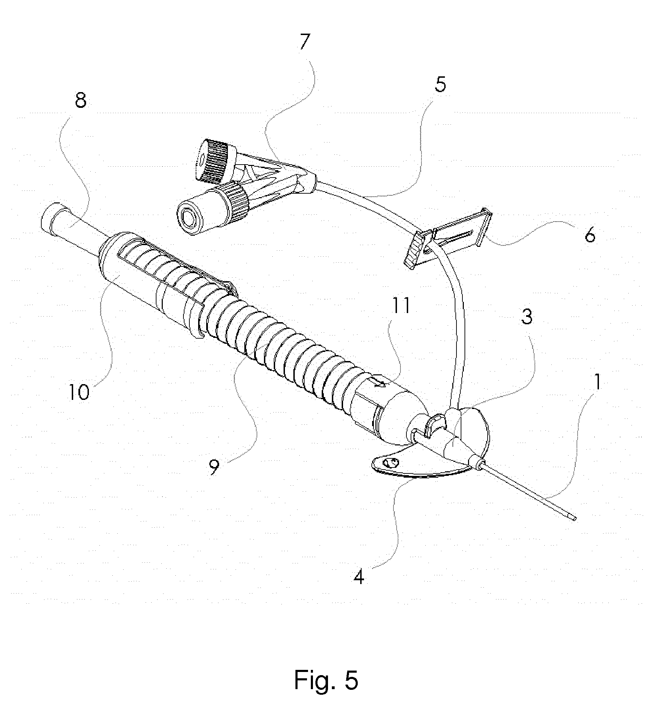

The CPIVCSS in its safety stage is shown in the isometric view in Figure 5. Once the catheter tube (1) is correctly placed in the circulatory system of the patient, the technician in charge of the procedure, holding the CPIVCSS in place in the affixing body (11) pulls the mounting body (10) away from the catheterization area which causes the flexible tube (9) to expand and the piercing cannula (2) withdraws from the inside of the catheter tube (1), from the patient's vein, from the catheter hub (3) and the hub seal (12).

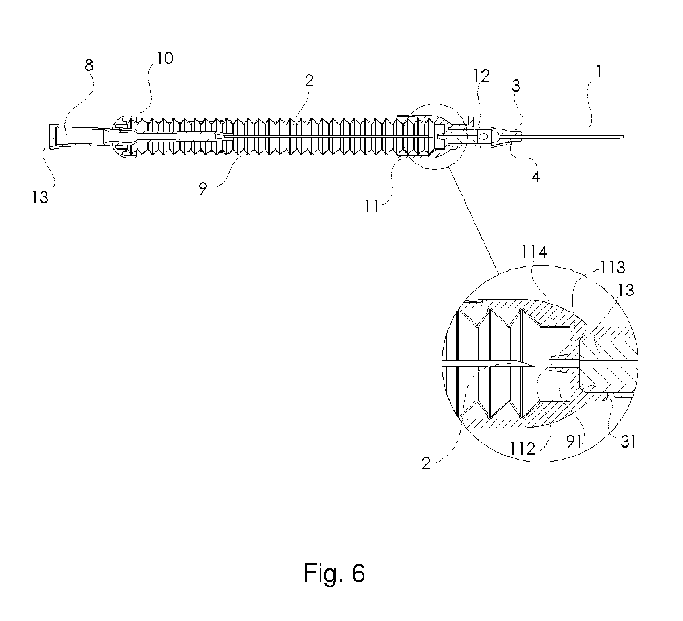

In Figure 6 we can see how the end of the piercing cannula (2) passes through the opening in the affixing body (112) being retained by the hatch of the affixing body (113) which makes the passage of the piercing cannula (2) back through the opening in the affixing body (112) very complicated, thus disabling the sharp tips of the piercing cannula (2) and encapsulating the whole within the flexible billows tube (9) which has expanded. The flexible billows tube is attached to the affixing body (11) and the mounting body (10) by means of bonding collars (114) in the joining and sticking cavities (91).

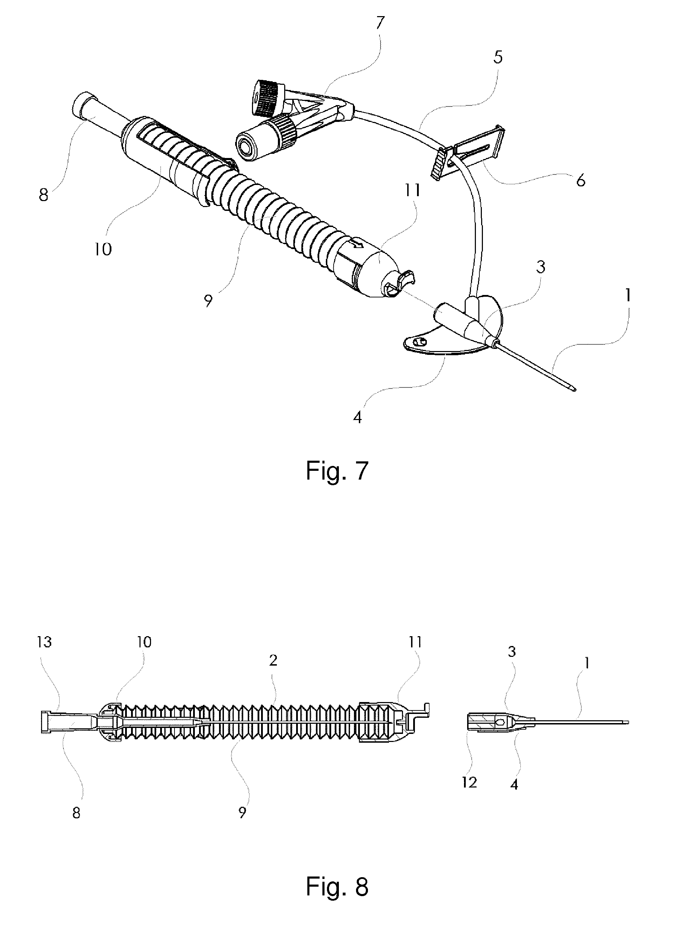

Once the flexible billows tube (9) has expanded and the piercing cannula (2) is encapsulated and the sharp ends of the affixing body (11) disabled, the technician in charge may proceed to disconnect the activated safety system of the closed peripheral intravenous catheter as shown in Figure 7. The closed peripheral intravenous catheter is firmly attached at the location of catheterization by means of affixing wings (4) and the system for administering medication is connected by means of the multi-piercing connector (7). The multi-piercing connector does not permit the entrance of foreign objects and micro-organisms inside the flow tube (5) and thus not inside the catheter hub (3), catheter tube and the patient's circulatory system either since the multi-piercing connectors only open when they are connected to the system for the administration of medication and thus the danger of infection in the area of catheterization is reduced.

Once the closed peripheral intravenous catheter is installed in the patient's circulatory system, disposing of the puncturing cannula (2) which is now contaminated with blood and other bodily fluids from the patient is begun. The piercing cannula remains encapsulated within the flexible billows tube (9) and its sharp ends are blocked by the affixing body (11) in order to avoid any possible accidental puncture wounds to medical personnel and contamination with the blood remaining in the used piercing cannula (2).

### Reference

[1.] A. M. Rivera , K. W. Strauss , A. Van Zundert, and E. Mortier, The history of peripheral intravenous catheters : How little plastic tubes revolutionized medicine, Acta Anaesth. Belg., 2005, 56, 271-282.

The invention has been sufficiently described so that a person with knowledge of the field can reproduce and obtain the results we mention for the present invention. However, anyone with knowledge of the field of the present invention is capable of making modifications not described in the present application, and if, for the application of these modifications in the determined structure or manufacturing process it is necessary to use the material claimed in the following claims, said structure should be considered within the scope of the invention.

Having sufficiently described the invention, the content of the following claim clauses is considered novel and therefore our property.

## Claims

1. A closed peripheral intravenous catheter with safety system CPIVCSS, with a safety system for avoiding accidental puncture wounds which consists of: a flexible billows tube (3) with an affixing body (2) at one of the long ends and a mounting body (5) on the other end, **characterized by** the affixing body having on the far end a cannula carrier which acts as a catheter cube or hub (3) with a side shunt hydraulically connected to a closed flow tube system (5), on whose free end is hydraulically connected at least a multi-piercing connector (7); on the far longitudinal end said cannula carrier or catheter cube or hub is attached to the catheter (1) for administering fluids and medication in a way that avoids exposure to the outside environ and reduces the possibility of the growth of infectious micro-organisms inside the catheter and in the area of catheterization.

2. A closed peripheral intravenous catheter with safety system CPIVCSS, as claimed in the previous claim, **characterized by** besides consisting of a mounting body (10) which is attached to a flexible billows tube (9) and to the affixing body (11) to which is connected the catheter hub (3); the piercing cannula (2) is attached to the mounting body (10) and passes concentrically through the flexible billows tube (9) and the affixing body (11), penetrating the hub seal (12) to cross the catheter hub (3) and remain housed inside the catheter tube (1) which is attached to the catheter hub (3) at one end, and on the side of the catheter hub (3) a flow tube (5) is attached which on its other end is attached to a multi-piercing connector (7) and above the flow tube (5) is mounted the safety flow lock (6).

3. A closed peripheral intravenous catheter with safety system CPIVCSS, as claimed in Claim 1, also **characterized by** consisting of: a safety system made up of a retractable piercing cannula (2) in a manual mechanism of flexible billows tube (9) to prevent re-use, accidental puncture wounds and assure safe disposal.

4. A closed peripheral intravenous catheter with safety system CPIVCSS, as claimed in Claim 1, also **characterized by** consisting of a catheter cube or hub (3) with affixing wings (4) to prevent movement of the closed peripheral intravenous catheter in the initial position of catheterization.

5. A closed peripheral intravenous catheter with safety system CPIVCSS, as claimed in Claim 1, also **characterized by** there being between the affixing body (11) and the catheter hub (3), on the inside, a hub seal (12) which allows the passage of the piercing cannula (2) but does not allow the passage of fluid or blood when the cannula is withdrawn and is placed within the billows tube (6).

6. A closed peripheral intravenous catheter with safety system CPIVCSS, as claimed in Claim 2, also **characterized by** the safety flow valve (6) in the flow tube (5) consisting of a plate with a longitudinal center slit with a dimension that varies from one end to the other, one end being larger in diameter than the outside of the flow tube (5) and the other end being smaller.
